# EUROPÄISCHE PATENTSCHRIFT

(11) **EP 2 533 826 B1**
(45) Veröffentlichungstag und Bekanntmachung des Hinweises auf die Patenterteilung: **17.01.2018**
(21) Anmeldenummer: 11703153.4
(22) Anmeldetag: 09.02.2011
(51) Int. Cl.: A61M 1/16

(54) **MEDIZINISCHE FUNKTIONSEINRICHTUNG, BEHANDLUNGSVORRICHTUNG SOWIE VERFAHREN**
MEDICAL FUNCTIONAL UNIT, TREATMENT DEVICE AND METHOD
INSTALLATION MÉDICALE, DISPOSITIF DE TRAITEMENT ET PROCÉDÉS

(30) Priorität: 10.02.2010 DE 102010007464
(43) Veröffentlichungstag der Anmeldung: 19.12.2012
(73) Patentinhaber: Fresenius Medical Care Deutschland GmbH, 61352 Bad Homburg (DE)
(72) Erfinder: HEIDE, Alexander, 65817 Eppstein (DE); KLEWINGHAUS, Jürgen, 61440 Oberursel (DE); PARTENFELDER, Robin, 61440 Oberusel (DE); PETERS, Arne, 61352 Bad Homburg (DE); WIKTOR, Christoph, 63571 Gelnhausen (DE)
(74) Vertreter: Bobbert & Partner Patentanwälte PartmbB
(86) Internationale Anmeldenummer: PCT/EP2011/000599
(87) Internationale Veröffentlichungsnummer: WO 2011/098265

(56) Entgegenhaltungen:
- EP-A1- 0 905 379
- WO-A1-2009/094183
- WO-A1-2009/110652
- WO-A1-2009/127627
- WO-A1-2011/045167
- WO-A2-2005/042065
- WO-A2-2009/026060
- DE-A1-102007 010 112
- JP-A- 2003 062 065
- JP-A- 2004 144 070
- US-A- 5 350 357
- US-A1- 2003 204 162
- US-A1- 2004 019 313
- US-A1- 2008 217 245
- US-A1- 2009 095 679
- US-A1- 2009 120 864
- US-A1- 2009 299 272
- US-B1- 6 227 820
- US-B1- 6 439 845
- US-B2- 6 814 547

## Beschreibung

Die vorliegende Erfindung betrifft eine medizinische Funktionseinrichtung gemäß Anspruch 1. Sie betrifft ferner eine Behandlungsvorrichtung gemäß Anspruch 12.

Medizinische Funktionseinrichtungen sind in der Regel dazu vorgesehen, verschiedene Funktionen bei der Durchführung einer medizinischen Behandlung zu erfüllen. Dabei können sie dazu ausgelegt sein, medizinische Fluide wie Blut aufzunehmen und von ihnen durchströmt zu werden.

Aus der DE 10 2007 010 112 A1 ist eine Vorrichtung für den Stoff- und/oder Energieaustausch zwischen zwei Medien bekannt.

Aus der US 2008/0217245 A1 ist ein Gerät zur kontinuierlichen Nierenersatztherapie bekannt.

Aus der WO 2009/094183 A1 ist ein automatisches Flüssigkeitsleitungs-Verbindungsgerät und ein Verfahren für ein medizinisches Behandlungssystem bekannt.

Eine Aufgabe der vorliegenden Erfindung ist, eine weitere medizinische Funktionseinrichtung vorzuschlagen.

Diese Aufgabe wird durch eine medizinische Funktionseinrichtung mit den Merkmalen des Anspruchs 1 gelöst.

Die medizinische Funktionseinrichtung gemäß der vorliegenden Erfindung weist wenigstens ein erstes Fluidsystem, geeignet und/oder vorgesehen zum Aufnehmen wenigstens eines medizinischen Fluids, wenigstens eine erste Fördereinrichtung zum Fördern des medizinischen Fluids, wenigstens ein zweites Fluidsystem, geeignet und/oder vorgesehen zum Aufnehmen wenigstens eines Arbeitsfluids, und wenigstens eine zweite Fördereinrichtung, geeignet und/oder vorgesehen zum Antreiben der wenigstens ersten Fördereinrichtung auf.

Die erste Fördereinrichtung ist dabei vorzugsweise derart angeordnet, um mittels des Arbeitsfluids betätigt oder angetrieben oder mit Energie versorgt zu werden. Die zweite Fördereinrichtung ist als Zentrifugalpumpe oder als Kreiselpumpe ausgestaltet.

Vorteilhafte Weiterbildungen der erfindungsgemäßen medizinischen Funktionseinrichtung sind Gegenstand der Unteransprüche und/oder der folgenden Ausführungen und Ausführungsformen.

Bei allen folgenden Ausführungen ist der Gebrauch des Ausdrucks "kann sein" bzw. kann haben" usw. synonym zu "ist vorzugsweise" bzw. "hat vorzugsweise" usw. zu verstehen und soll jeweils unabhängig voneinander erfindungsgemäße Ausführungsformen erläutern.

Die erfindungsgemäße medizinische Funktionseinrichtung kann eine externe oder eine interne medizinische Funktionseinrichtung sein. Dabei wird unter "extern" im Sinne der vorliegenden Erfindung eine Komponente verstanden, die nicht in der Behandlungsvorrichtung vorliegt. Ein Beispiel für eine externe medizinischen Funktionseinrichtung ist eine Wegwerf-Blutkassette. Unter "intern" im Sinne der vorliegenden Erfindung wird eine Komponente verstanden, die in der Behandlungsvorrichtung vorliegt, also intern ist oder innerhalb angeordnet ist, ggf. auch austauschbar.

Die erfindungsgemäße medizinische Funktionseinrichtung kann für die Blutbehandlung (z.B. Hämodialyse, Hämofiltration, Hämodiafiltration) und die Mehrfachinfusionstechnik geeignet und vorgesehen sein. Sie kann aber auch zur Peritonealdialyse geeignet und vorgesehen sein (z.B. APD = Automated Peritoneal Dialysis, APD-Gerät).

Der Begriff "Fluidsystem", wie er hierin verwendet wird, bezeichnet ein System oder eine Anordnung, welche(s) dazu geeignet und vorgesehen ist, Fluide aufzunehmen.

Das Fluidsystem bzw. die Fluidsysteme sind vorzugsweise, insbesondere im Wesentlichen oder vollständig, Teil der erfindungsgemäßen medizinischen Funktionseinrichtung. Beispielsweise können die Fluidsysteme integral mit der medizinischen Funktionseinrichtung ausgestaltet sein bzw. in die medizinische Funktionseinrichtung integriert sein.

Das Fluidsystem bzw. die Fluidsysteme können Leitungen, Schläuche, Schlauchsysteme, Kanäle, Ventile, Drosseln, Filter, Sensoren, Kammern, Ausbuchtungen, Einrichtungen oder Räume oder Bereiche zum Speichern oder Vorhalten oder Zurückhalten von Fluiden sowie Steuer- oder Regeleinrichtungen zum Steuern oder Regeln eines Strömungsdurchflusses der Fluide und dergleichen aufweisen oder hieraus bestehen.

Die Fluidsystem können mit einem (übergeordneten) Fluidkreislauf verbunden oder verbindbar sein. Sie können Abschnitte oder Bereiche des Fluidkreislaufs bilden. Die Fluidsysteme können in den Fluidkreislauf eingebunden sein.

Ein "erstes Fluidsystem" ist erfindungsgemäß dazu ausgelegt und vorgesehen, wenigstens ein medizinisches Fluid aufzunehmen und von diesem durchströmt zu werden.

Bei einem "medizinischen Fluid" kann es sich um eine Flüssigkeit, ein Gas, eine flüssige Zubereitung, eine Kombination oder Mischung verschiedener Flüssigkeiten und/oder Gase oder dergleichen handeln. Das medizinische Fluid kann die Peritoneal-Lösung (PD-Lösung) aus Beuteln (APD Solution Bags) sein. Dieses Fluid wird üblicherweise über ein und denselben Katheter in den Bauchraum des Patienten eingebracht und aus diesem wieder entfernt.

Das medizinische Fluid ist in bestimmten erfindungsgemäßen Ausführungsformen dazu vorgesehen, während der Verwendung der vorliegenden Erfindung oder eines erfindungsgemäßen Gegenstandes mit dem Blut oder einem anderen Bestandteil des Körpers, einem Gewebe oder Organ des Patienten, extrakorporal oder intrakorporal, in Kontakt zu gelangen oder sich mit diesem zu vermischen.

Ein "zweites Fluidsystem" ist erfindungsgemäß dazu ausgelegt und vorgesehen, wenigstens ein Arbeitsfluid aufzunehmen und von diesem durchströmt zu werden.

Bei einem "Arbeitsfluid" kann es sich um eine Flüssigkeit, ein Gas, eine flüssige Zubereitung, eine Kombination oder Mischung verschiedener Flüssigkeiten und/oder Gase oder dergleichen handeln.

In bestimmten erfindungsgemäßen Ausführungsformen sind sowohl das Arbeitsfluid als auch das medizinische Fluid jeweils - gleiche oder voneinander verschiedene - Flüssigkeiten oder Flüssigkeitsgemische. In diesen Ausführungsformen betrifft die Erfindung kein pneumatisches System, weist kein pneumatisches System auf oder ist nicht als solches ausgestaltet.

Der Begriff "Fördereinrichtung", wie er hierin verwendet wird, bezeichnet eine Einrichtung, welche zum Fördern wenigstens eines Fluid innerhalb eines Fluidsystems vorgesehen und konfiguriert ist.

Die erste und/oder die zweite Fördereinrichtung sind vorzugsweise mit der medizinischen Funktionseinrichtung verbunden oder mit dieser verbindbar ausgestaltet oder sind Bestandteil hiervon.

Die erste und/oder die zweite Fördereinrichtung können jeweils kraft- und/oder form- und/oder stoffschlüssig mit der medizinischen Funktionseinrichtung verbunden sein.

Vorzugsweise sind die erste Fördereinrichtung und/oder die zweite Fördereinrichtung in die medizinische Funktionseinrichtung integriert oder integral mit dieser verbunden.

Die erste Fördereinrichtung kann dazu eingesetzt werden, medizinisches Fluid von einem Äußeren der medizinischen Funktiönseinrichtung in ein Inneres des ersten Fluidsystems zu fördern und/oder umgekehrt.

Der Begriff "Äußeres der medizinischen Funktionseinrichtung", wie er hierin verwendet wird, bezeichnet einen Bereich oder Abschnitt eines Fluidkreislaufs, wie eines extrakorporalen Blutkreislaufs, welcher nicht Teil der medizinischen Funktionseinrichtung ist.

Die zweite Fördereinrichtung kann dazu vorgesehen sein und/oder dazu eingesetzt werden, Arbeitsfluid innerhalb des zweiten Fluidsystems zu fördern und/oder Arbeitsfluid von einem Äußeren der medizinischen Funktionseinrichtung in ein Inneres des zweiten Fluidsystems zu fördern und/oder umgekehrt. Die zweite Fördereinrichtung kann dazu in oder an dem zweiten Fluidsystem angeordnet sein.

Durch Fördern des Arbeitsfluids innerhalb des zweiten Fluidsystems kann eine Betätigung der ersten Fördereinrichtung erreicht werden.

Der Begriff "Betätigung" der ersten Fördereinrichtung, wie er hierin verwendet wird, bezeichnet ein Antreiben bzw. Betreiben oder Ansteuern der ersten Fördereinrichtüng. Dabei kann es sich beispielsweise um ein Versorgen der ersten Fördereinrichtung mit Energie, z.B. Strömungsenergie (mechanische Leistung), und/oder ein Beaufschlagen der ersten Fördereinrichtung mit Druck oder dergleichen handeln. Die erste Fördereinrichtung kann beispielsweise mit Arbeitsfluid gefüllt und wieder entleert werden. Die Betätigung der ersten Fördereinrichtung kann hydraulisch erfolgen.

Das Arbeitsfluid kann zur Signal-, Kraft- und/oder Energieübertragung eingesetzt werden. Auch die medizinischen Fluide des ersten Fluidkreislaufs können zur Signal-, Kraft- und/oder Energieübertragung eingesetzt werden.

Die erste Fördereinrichtung kann durch Betätigung der zweiten Fördereinrichtung betätigt werden. Die Betätigung der ersten Fördereinrichtung kann durch die Wirkung der zweiten Fördereinrichtung hervorgerufen bzw. ausgelöst werden.

Die Betätigung oder Ansteuerung der ersten Fördereinrichtung kann lediglich unterstützend oder ausschließlich durch Betätigung der zweiten Fördereinrichtung erfolgen.

Das erste Fluidsystem und das zweite Fluidsystem können zusammen mit der (den) ersten Fördereinrichtung(en) und der (den) zweiten Fördereinrichtung(en) ein hydraulisches System oder einen Hydraulikkreislauf bilden.

Es können mehrere Fluidleitungen für mehrere medizinische Flüssigkeiten oder Teilmengen der medizinischen Flüssigkeiten mit dementsprechend mehreren separaten ersten Fördereinrichtungen vorgesehen sein.

In einer bevorzugten Ausführungsform ist wenigstens eine erste Fördereinrichtung mit einer Druckseite und/oder einer Saugseite der zweiten Fördereinrichtung verbunden oder verbindbar angeordnet.

Die erste Fördereinrichtung kann mit einer Druckleitung und/oder einer Saugleitung des zweiten Fluidsystems verbunden oder verbindbar angeordnet sein. Es kann eine abzweigende Leitung von der Druckseite und/oder von der Saugseite der zweiten Fördereinrichtung zu der ersten Fördereinrichtung gelegt sein. Die abzweigende(n) Leitung(en) kann (können) mit der Druckleitung und/oder der Saugleitung des zweiten Fluidsystems verbunden sein bzw. von diesen abzweigen.

Während des Betriebs der zweiten Fördereinrichtung kann so eine Druckdifferenz des Arbeitsfluids zwischen der Druckseite und der Saugseite der zweiten Fördereinrichtung zum Antreiben der ersten Fördereinrichtung(en) verwendet werden.

Der Begriff "wenigstens eine erste Fördereinrichtung" bzw. "wenigstens eine zweite Fördereinrichtung", wie er hierin verwendet wird, meint, dass wenigstens eine, vorzugsweise jedoch eine Mehrzahl oder Vielzahl an ersten bzw. zweiten Fördereinrichtungen vorgesehen ist.

Was im Folgenden zur ersten Fördereinrichtung gesagt wird, trifft erfindungsgemäß auch für die zweite Fördereinrichtung zu.

Die ersten Fördereinrichtungen können gleichartig bzw. vom gleichen oder identischen Typ sein. Sie können baugleich sein.

Die ersten Fördereinrichtungen können verschiedenartig sein.

Die ersten Fördereinrichtungen können nach verschiedenen Funktions- oder Betriebs- oder Wirkweisen arbeiten.

Eine Mehr- oder Vielzahl an ersten Fördereinrichtungen kann gleichartig bzw. von einem ersten Typ sein. Eine andere Mehr- oder Vielzahl an ersten Fördereinrichtungen kann als Fördereinrichtung eines anderen Typs oder mehrerer anderer Typen ausgestaltet sein.

Die ersten Fördereinrichtungen können mit Hilfe von geeigneten Steuer- oder Regeleinrichtungen gesteuert oder geregelt werden. Es kann einer Mehr- oder Vielzahl solcher Steuer- oder Regeleinrichtungen vorgesehen sein.

Die ersten Fördereinrichtungen können gemeinsam bzw. mittels einer einzigen Steuer- oder Regeleinrichtung gesteuert oder geregelt bzw. reguliert werden.

Jede erste Fördereinrichtung kann von einer anderen (separaten) Steuer- oder Regeleinrichtung gesteuert oder geregelt werden.

Die ersten Fördereinrichtungen können von verschiedenen und/oder unterschiedlichen Steuer- oder Regeleinrichtungen mit beispielsweise unterschiedlichem Wirkprinzip gesteuert oder geregelt werden.

Ein Teil der ersten Fördereinrichtungen kann gemeinsam geregelt werden, ein anderer Teil separat.

Die ersten Fördereinrichtungen können derart gesteuert oder geregelt werden, dass sie gleichzeitig und/oder in gleichem Ausmaß oder nicht gleichzeitig und/oder in verschiedenem Ausmaß oder auch gar nicht fördern können.

Geeignete Steuer- oder Regeleinrichtungen schließen Ventile bzw. Ventileinrichtungen, Durchflussregel- oder - steuereinrichtungen, Einrichtungen zum Veranlassen oder Unterstützen eines Eintragens und/oder eines Austragens von Fluiden, wie Düsen, Venturi-Düsen, Tüllen, Ports, Stutzen, Durchflusssperreinrichtungen wie Klemmen, Absperrhähne, Schikanen, die Strömungsgeschwindigkeit bzw. die Durchflussmenge regulierende oder verändernde Einrichtungen wie Strömungsschnellen und dergleichen ein.

Die ersten Fördereinrichtungen können innerhalb des gleichen Fluidsystems angeordnet sein.

Die ersten Fördereinrichtungen können in Abschnitten oder Bereichen eines (übergeordneten) ersten Fluidsystems der medizinischen Funktionseinrichtung vorgesehen sein. Sie können in nebengeordneten Fluidsystemen und/oder in Abzweigungen bzw. Verzweigungen des ersten Fluidsystems angeordnet sein.

Die ersten Fördereinrichtungen können jeweils in einem separaten, d.h. für jede Fördereinrichtung eigenen, Fluidsystem angeordnet sein.

Einzelne Fluidsysteme können räumlich und/oder funktionell voneinander getrennt sein.

Die ersten Fördereinrichtungen können gleiche oder identische Fluide fördern. Sie können voneinander verschiedene bzw. unterschiedliche Fluide fördern. Bei den mittels der ersten Fördereinrichtungen geförderten Fluiden kann es sich jeweils um medizinische Fluide handeln. Ein Teil der ersten Fördereinrichtungen kann jedoch auch nicht-medizinische Fluide fördern.

In bevorzugten Ausführungsformen sind erste Fördereinrichtungen in Parallelschaltung und/oder in Reihenschaltung mit der Druckseite und/oder der Saugseite der zweiten Fördereinrichtung oder zwischen Druck- und Saugseite verbunden oder verbindbar angeordnet.

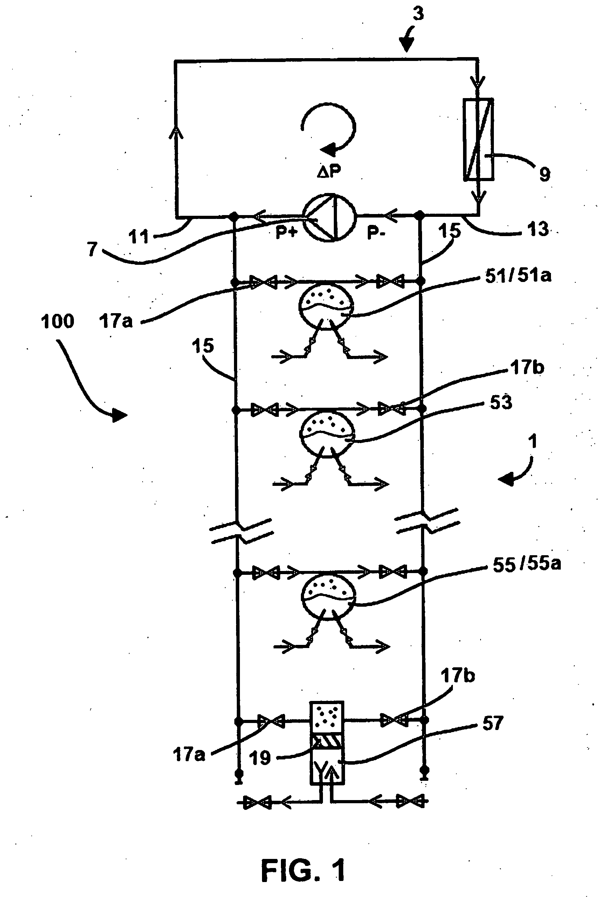

Fig. 1 zeigt beispielhaft eine solche Anordnung.

In einer weiteren bevorzugten Ausführungsform ist die erste Fördereinrichtung als Verdrängerpumpe ausgestaltet.

Geeignete Verdrängerpumpen schließen z.B. Membranpumpen, Kolbenpumpen, Schlauchpumpen, peristaltische Pumpen ein.

Membranpumpen können eine Kammer zur Förderung der medizinischen Fluide aufweisen, die durch eine Pumpenmembran vom Arbeitsfluid getrennt sind. Das Arbeitsfluid dient der Verschiebung der Membran zur Erzielung einer Pumpwirkung.

Die Zentrifugalpumpe oder Kreiselpumpe als zweite Fördereinrichtung kann vorteilhaft derart ausgestaltet und konfiguriert sein, dass sie einen hohen Volumenstrom bei niedrigen Drücken und/oder einen niedrigen Volumenstrom bei hohen Drücken und/oder einen hohen Druck ohne Volumenstrom bereitstellt. Dabei hängt die Druckdifferenz von der Drehzahl ab.

Der maximale Druck bei einer Zentrifugalpumpe kann über die Drehzahl eingestellt werden, so dass die maximale Druckbelastung auf das gesamte hydraulische System vorteilhaft (sehr) exakt definierbar sein kann.

In einer weiteren bevorzugten Ausführungsform weist die zweite Fördereinrichtung wenigstens einen drehenden bzw. rotierenden Abschnitt bzw. einen Rotationsabschnitt zum Fördern des Arbeitsfluids auf.

Der Rotationsabschnitt kann ausschließlich bzw. vollständig oder ergänzend magnetisch gelagert sein. Die Lagerung kann vollständig oder ergänzend mittels einer Achse erfolgen. Die Lagerungsart kann unabhängig von der Art der Übertragung der Antriebsleistung sein.

Der Rotationsabschnitt kann in einem Inneren der zweiten Fördereinrichtung angeordnet sein.

Der Rotationsabschnitt kann ein Impeller sein.

Die zweite Fördereinrichtung kann als Impellerpumpe ausgestaltet sein.

In einer weiteren bevorzugten Ausführungsform ist der Rotationsabschnitt konfiguriert, um mittels eines externen Antriebs magnetisch angetrieben zu werden. Der Antrieb könnte alternativ auch mechanisch, z.B. mittels einer Antriebswelle erfolgen.

Der Begriff "externer Antrieb", wie er hierin verwendet wird, bezeichnet einen Antrieb des Rotationsabschnitts, welcher nicht Teil der medizinischen Funktionseinrichtung ist.

Der externe Antrieb kann an einer Vorrichtung angeordnet sein. Der externe Antrieb kann einen Teil der Vorrichtung bilden.

Die Vorrichtung kann dazu vorgesehen und konfiguriert sein, um funktionell mit der erfindungsgemäßen medizinischen Funktionseinrichtung gekoppelt zu werden.

Die magnetische Antriebskraft oder -wirkung kann mit Hilfe von Magneten erzielt werden. Sie kann mit Hilfe von stromdurchflossenen Leitern erzielt werden. Beispielsweise können stromdurchflossene Spulen verwendet werden.

Die zweite Fördereinrichtung kann eine magnetisch gelagerte Zentrifugalpumpe sein, wie sie kommerziell am Markt erhältlich ist.

Eine solche magnetisch gelagerte Zentrifugalpumpe kann den Vorteil bieten, dass keine mechanische und/oder elektrische Schnittstelle zur Maschine bzw. der Behandlungsvorrichtung erforderlich ist und/oder keine Fluide von der Maschine zur Pumpe übertragen werden müssen. Dies kann eine besonders günstige Herstellung der erfindungsgemäßen Funktionseinrichtung erlauben. Dies kann ferner die bei derartigen Schnittstellen mögliche Kontaminationsgefahr verringern.

In einer weiteren bevorzugten Ausführungsform sind das erste Fluidsystem und das zweite Fluidsystem fluiddicht voneinander getrennt ausgestaltet.

Das erste Fluidsystem und das zweite Fluidsystem können im Wesentlichen oder vollständig fluiddicht voneinander getrennt sein.

Der Begriff "fluiddicht", wie er hierin verwendet wird, bezeichnet einen Zustand, in dem ein Übergang oder Übertritt von Fluiden zwischen dem ersten Fluidsystem und dem zweiten Fluidsystem im Wesentlichen oder vollständig ausgeschlossen ist.

Dies kann bedeuten, dass kein Arbeitsfluid aus dem zweiten Fluidsystem in das erste Fluidsystem gelangen kann. Ebenso kann es bedeuten, dass kein medizinisches Fluid aus dem ersten Fluidsystem in das zweite Fluidsystem gelangen kann.

Das erste Fluidsystem und/oder das zweite Fluidsystem können in sich geschlossene Fluidsysteme darstellen.

Dies kann den Vorteil bieten, dass eine Vermischung von Arbeitsfluid mit medizinischem Fluid im Wesentlichen oder vollständig vermieden werden kann. Eine mögliche Kontaminationsgefahr für die zu behandelnden medizinischen Fluide kann so vorteilhaft verringert oder ausgeschlossen werden.

Bei den medizinischen Fluiden kann es sich beispielsweise um Blut, Dialysierflüssigkeit, Substituatflüssigkeit, Medikamente, Medikamentenzubereitungen sowie Mischungen oder Kombinationen derselben handeln.

Unter einer "Medikamentenzubereitung", wie sie hierin verwendet wird, können Lösungen, Suspensionen, Emulsionen, Extrakte und dergleichen von Medikamenten in Kombination mit geeigneten Lösungsmitteln, Exzipienten und dergleichen verstanden werden.

Medikamente schließen z.B. Antikoagulationsmittel wie Heparin und Citrat sowie Koagulantien wie Calcium, z.B. in Form einer Ci-Ca-Antikoagulation, und dergleichen ein. Es kann vorgesehen sein, die Medikamente in den extrakorporalen Blutkreislauf zu infundieren.

Das Arbeitsfluid kann ausgewählt sein aus Dialysierflüssigkeit, Substituatflüssigkeit, steriler Druckluft, Öl, Hydrauliköl, Wasser, aufbereitetes Wasser (Permeat) aus einer Umkehrosmoseanlage oder -quelle (RO-Anlage) oder andere Flüssigkeiten, sowie Mischungen oder Kombinationen derselben.

Die Substituatflüssigkeit kann steril sein. Die Substituatflüssigkeit kann eine isotone Kochsalzlösung wie z.B. 0.9%-ige NaCl-Lösung sein.

Wenn Dialysierflüssigkeit und/oder Substituatflüssigkeit sowohl als medizinisches Fluid als auch als Arbeitsfluid eingesetzt werden, können sie der gleichen Quelle entnommen werden. Zu diesem Zweck können die Dialysierflüssigkeit und/oder die Substituatflüssigkeit aus dem gleichem Vorrat ständig zirkuliert werden. Es ist jedoch ebenso möglich, eine Teilmenge an Dialysierflüssigkeit und/oder Substituatflüssigkeit aus dem Vorrat abzuzweigen und nur zur zum Betätigen der ersten Fördereinrichtungen (d.h. zur "Energieerzeugung") zu verwenden.

Die Verwendung von Dialysierflüssigkeit und/oder Substituatflüssigkeit als Arbeitsfluid kann vorteilhaft dazu beitragen, eine Sicherheit des hydraulischen Antriebssystems der erfindungsgemäßen medizinischen Funktionseinrichtung zu erhöhen.

So kann beispielsweise selbst in einem an sich auszuschließenden Fehlerfall einer Kontamination des zu behandelnden medizinischen Fluids vorteilhaft vorgebeugt werden.

In einer weiteren bevorzugten Ausführungsform ist die medizinische Funktionseinrichtung als Disposable-Kassette und/oder als Schlauchsatz ausgestaltet.

Der Begriff "Disposable-Kassette", wie er hierin verwendet wird, bezeichnet eine Einrichtung, welche - beispielsweise im Bereich der Medizin oder Medizintechnik - als Einmalartikel oder Wegwerfartikel ausgestaltet ist bzw. als solcher eingesetzt wird.

Die medizinische Funktionseinrichtung kann als Disposable-Kassette zur extrakorporalen Blutbehandlung, insbesondere als Blutkassette, die Teil eines extrakorporalen Blutkreislaufs ist, und/oder als Dialysatkassette, die Teil eines Dialysatkreislaufs ist oder hierzu in ihrem Normalgebrauch vorgesehen ist, ausgestaltet sein.

Die Disposable-Kassette kann ganz oder teilweise aus einem Hartteil bestehen. Sie kann aus einem Kunststoffmaterial hergestellt sein. Die Disposable-Kassette kann mittels eines Spritzgussverfahrens hergestellt sein.

Bestandteile der Kassette, wie Fluidsysteme, z.B. Leitungen, Kanäle, Kammern, Ventile, Drosseln und dergleichen, können während der Herstellung der Disposable-Kassette gebildet werden. Andere Bestandteile der Kassette, wie beispielsweise erste und/oder zweite Fördereinrichtungen können während der Herstellung der Disposable-Kassette mit dieser integral ausgestaltet werden und/oder während oder nach Fertigstellung der Disposable-Kassette mit dieser kraft- und/oder form- und/oder stoffschlüssig verbunden werden.

Die Aufgabe der vorliegenden Erfindung wird ferner durch eine Behandlungsvorrichtung gemäß Anspruch 13 gelöst. Alle mit der erfindungsgemäßen medizinischen Funktionseinrichtung erzielbaren Vorteile lassen sich ungeschmälert auch mit der erfindungsgemäßen Behandlungsvorrichtung erzielen.

Die erfindungsgemäße Behandlungsvorrichtung ist zum Behandeln medizinischer Fluide geeignet. Sie weist wenigstens eine Steuer- oder Regeleinrichtung auf, die dazu vorgesehen und/oder konfiguriert ist, um wenigstens eine zweite Fördereinrichtung einer medizinischen Funktionseinrichtung anzutreiben. Die Steuer- oder Regeleinrichtung ist in manchen erfindungsgemäßen Ausführungsformen vorgesehen und/oder konfiguriert, Ventile im ersten und/oder im zweiten Fluidsystem mittels geeigneter Aktoren zu steuern und/oder zu regeln.

Die Steuer- oder Regeleinrichtung kann computergestützt sein. Sie kann ein Mikroprozessor sein oder einen solchen aufweisen.

In einer bevorzugten Ausführungsform weist die Behandlungsvorrichtung eine Einrichtung auf, die dazu vorgesehen und konfiguriert ist, die zweite Fördereinrichtung über eine magnetische Antriebsschnittstelle anzutreiben.

Eine solche Einrichtung kann eine magnetische Wirkung oder Kraft erzeugen oder hervorrufen, welche zum Antreiben der zweiten Fördereinrichtung bzw. von Teilen oder Abschnitten derselben, eingesetzt werden kann. Die Einrichtung kann insbesondere dazu vorgesehen sein, auf den Rotationsabschnitt der zweiten Fördereinrichtung, wie einen Impeller, zu wirken.

Die Einrichtung kann beispielsweise als Magnet oder magnetisch wirkendes System und/oder als stromdurchflossene(r) Leiter, wie beispielsweise eine oder mehrere stromdurchflossene Spule(n), ausgestaltet, sein.

Die Behandlungsvorrichtung kann ferner weitere Schnittstellen aufweisen. Solche weiteren Schnittstellen können ebenfalls dazu vorgesehen sein, die zweite Fördereinrichtung bzw. Teile oder Abschnitte derselben anzutreiben. Sie können ebenfalls dazu vorgesehen und konfiguriert sein, Steuer- oder Regeleinrichtungen zum Ansteuern der ersten Fördereinrichtungen anzusteuern oder zu betätigen.

Die Behandlungsvorrichtung kann funktionell mit einer medizinischen Funktionseinrichtung, insbesondere einer erfindungsgemäßen medizinischen Funktionseinrichtung, koppelbar sein, zu ihrer Koppelung vorgesehen sein, oder eine solche aufweisen.

Um eine medizinische Behandlung durchzuführen, kann die Behandlungsvorrichtung ferner mit weiteren Einrichtungen koppelbar sein, zu ihrer Koppelung vorgesehen sein, oder derartige Einrichtungen aufweisen, wie beispielsweise einen extrakorporalen Blutkreislauf, Steuer- oder Regeleinrichtungen zum Ansteuern der Durchführung einer medizinischen Behandlung, Einrichtungen zum Anzeigen oder Darstellen von Zuständen und/oder Parametern der medizinischen Behandlung, wie Bildschirmen und dergleichen, Einrichtungen zum Bedienen bzw. Betätigen oder Ansteuern einer oder mehrerer Komponenten der Behandlungsvorrichtung, wie Tastaturen und dergleichen, um z.B. die Durchführung einer medizinischen Behandlung zu veranlassen, und dergleichen.

Die Behandlungsvorrichtung weist in manchen Ausführungsformen eine ebene oder unebene Ankoppelfläche auf, welche eine Schnittstelle zwischen der medizinischen Funktionseinrichtung und der Behandlungsvorrichtung ist. Die Ankoppelfläche kann Aktoren (z.B. zur Ventilbetätigung), Sensoren (z.B. Drucksensoren) und Ankoppelelemente (z.B. Verpressungsvorrichtungen) aufweisen.

Die Behandlungsvorrichtung kann beispielsweise als Blutbehandlungsvorrichtung, Mehrfachinfusionsvorrichtung, Peritonealdialysevorrichtung oder Infusionsvorrichtung ausgestaltet sein.

Alle mit der erfindungsgemäßen medizinischen Funktionseinrichtung erzielbaren Vorteile lassen sich ungeschmälert auch mit einem Verfahren erzielen.

Das Verfahren umfasst das Fördern wenigstens eines medizinischen Fluids unter Verwenden wenigstens einer erfindungsgemäßen medizinischen Funktionseinrichtung oder wenigstens einer erfindungsgemäßen Behandlungsvorrichtung.

In manchen Ausführungsformen umfasst das Verfahren einen oder mehrere der oben genannten oder aus dem oben Diskutierten herauslesbaren Verfahrensschritte oder Handlungen.

In einer bevorzugten Ausführungsform umfasst das Verfahren dabei das Antreiben wenigstens einer zweiten Fördereinrichtung zum Fördern wenigstens eines Arbeitsfluids innerhalb eines zweiten Fluidsystems zum Antreiben einer ersten Fördereinrichtung zum Antreiben eines medizinischen Fluids.

Das Verfahren kann das Ansteuern einer Vielzahl von Steuer- oder Regeleinrichtungen, um eine Vielzahl erster Fördereinrichtungen in Reihen- und/oder Parallelschaltung getrennt voneinander anzusteuern, umfassen.

Das Verfahren umfasst in manchen Ausführungsformen ein gleichzeitiges, gesteuertes oder geregeltes Betreiben mindestens einer Blutpumpe und mindestens einer Dialysatpumpe (wobei die Pumpen, auch im Folgenden, jeweils als erste Fördereinrichtungen zu verstehen sind).

Es umfasst in manchen Ausführungsformen ein gleichzeitiges, gesteuertes oder geregeltes Betreiben mindestens einer Pumpe für Heparin oder einer Heparin-haltigen Lösung.

Es umfasst in manchen Ausführungsformen ein gleichzeitiges, gesteuertes oder geregeltes Betreiben mindestens einer Pumpe für Citrat-haltige Lösung und/oder mindestens einer weiteren Pumpe für eine Calcium-haltige Lösung.

Das Verfahren umfasst in manchen Ausführungsformen ein gleichzeitiges, gesteuertes oder geregeltes Betreiben mindestens einer Substituatpumpe.

Es umfasst in manchen Ausführungsformen ein gleichzeitiges, gesteuertes oder geregeltes Betreiben mindestens einer Pre-Dilutionspumpe und/oder mindestens einer Post-Dilutionspumpe.

Es umfasst in manchen Ausführungsformen ein gleichzeitiges, gesteuertes oder geregeltes Betreiben mindestens einer Pumpe für die Infusion eines Medikaments in den extrakorporalen Blutkreislauf.

Es umfasst in manchen Ausführungsformen ein Steuern oder Regeln mindestens eines Ventils in den Leitungen für das Arbeitsfluid auf der Eintritts- und/oder der Austrittsseite der Pumpen.

Die vorliegende Erfindung stellt eine medizinische Funktionseinrichtung, z.B. in Form einer medizinischen Einmalkassette bereit. Diese kann aufgrund ihrer erfindungsgemäßen Ausgestaltung vorteilhaft einen besonders hohen Integrationsgrad aufweisen.

Die erfindungsgemäße medizinische Funktionseinrichtung kann vorteilhaft eine autarke Einheit bzw. ein autarkes System darstellen. Durch Antreiben der ersten Fördereinrichtungen mittels durch die zweite Fördereinrichtung geförderten Arbeitsfluids kann die medizinische Funktionseinrichtung vorteilhaft die zum Betrieb aller ersten Fördereinrichtungen sowie gegebenenfalls weiterer zur medizinischen Behandlung eingesetzten Fördereinrichtungen, wie beispielsweise einer Blutpumpe und/oder einer Fördereinrichtung einer Bilanzkammer, notwendige Energie selbst erzeugen.

Da das zu applizierende oder zu behandelnde medizinische Fluid in keiner direkten Fluid- oder Wirkverbindung mit der Behandlungsvorrichtung steht, kann vorteilhaft eine Kontaminationsgefahr der zu applizierenden oder zu behandelnden medizinischen Fluide verringert werden. Die medizinische Funktionseinrichtung der vorliegenden Erfindung kann eine hermetisch verschlossene Disposable-Kassette darstellen. Auf diese Weise kann sie vorteilhaft einen hohen Schutz der zu applizierenden oder zu behandelnden medizinischen Fluide bieten.

Da sowohl die ersten Fördereinrichtungen als auch die zweite Fördereinrichtung in die medizinische Funktionseinrichtung integriert sind, kann es vorteilhaft möglich sein, ein vereinfachtes Hygiene-Konzept für den Einsatz der medizinischen Funktionseinrichtung bereitzustellen. Da die erfindungsgemäße medizinische Funktionseinrichtung mit all ihren Komponenten als Einmalteil bzw. Disposable ausgeführt sein kann, kann vorteilhaft eine Reinigung und/oder Sterilisierung einzelner Geräte- oder Bauteile der erfindungsgemäßen medizinischen Funktionseinrichtung entfallen. Somit kann die erfindungsgemäße medizinische Funktionseinrichtung als Ganzes vorteilhaft eine kostengünstige Einrichtung für medizinische Behandlungsverfahren bereitstellen.

Da bei einer solchen medizinischen Funktionseinrichtung alle Bauteile oder Komponenten, die in Fluidkontakt gelangen, nach einmaligem Gebrauch der erfindungsgemäßen medizinischen Funktionseinrichtung entsorgt werden, kann die Sicherheit und Hygiene des medizinischen Behandlungsverfahrens vorteilhaft weiter verbessert werden.

Die magnetische Antriebsschnittstelle zum Betreiben der zweiten Fördereinrichtung bzw. eines Rotationsabschnitts derselben kann vorteilhaft einen berührungslosen und/oder dichtungsfreien Antrieb der zweiten Fördereinrichtung bereitstellen. Auf diese Weise kann es vorteilhaft möglich sein, auf offene Schnittstellen zwischen der medizinischen Funktionseinrichtung und der Behandlungsvorrichtung verzichten zu können.

Somit kann es vorteilhaft möglich sein, einen besonders sicheren Betrieb der medizinischen Funktionseinrichtung sicherzustellen. Eine Kontaminationsgefahr des Arbeitsfluids kann somit vorteilhaft verringert und sogar ganz ausgeschlossen werden.

Bei,der erfindungsgemäßen medizinischen Funktionseinrichtung kann es vorteilhaft möglich sein, die erforderliche Antriebsenergie für eine Mehrzahl von ersten Fördereinrichtungen über nur eine einzige (berührungslose) Schnittstelle auf die Disposable-Kassette zu übertragen und dort mittels der Druckerhöhung oder -veränderung des Arbeitsfluids auf die Mehrzahl von ersten Fördereinrichtungen zu verteilen. Somit kann vorteilhaft auf eine Vielzahl von individuellen maschinenseitigen Antrieben für eine Mehrzahl erster Fördereinrichtungen verzichtet werden. Die medizinische Funktionseinrichtung gemäß der vorliegenden Erfindung kann somit vorteilhaft ein sehr einfaches, kostengünstiges und/oder sicheres Antriebskonzept bereitstellen.

Da nur eine einzige Antriebsschnittstelle zwischen der medizinischen Funktionseinrichtung und der Behandlungsvorrichtung erforderlich ist, kann die erfindungsgemäße Behandlungsvorrichtung vorteilhaft eine entsprechend einfache und technisch wenig aufwändige Maschinenoberfläche aufweisen.

Die Maschinenoberfläche kann vorteilhaft frei gestaltbar sein. Die Maschinenoberfläche kann vorteilhaft leicht zu reinigen sein. Auf eine Luftankopplung und/oder Rotorachsen für Schlauchrollenpumpen kann vorteilhaft verzichtet werden.

Da die ersten Funktionseinrichtungen in der erfindungsgemäßen medizinischen Funktionseinrichtung nicht einzeln, z.B. über eine Kopplung zu einer äußeren Vorrichtung wie einer Behandlungsvorrichtung, betrieben werden müssen, kann es vorteilhaft möglich sein, einen technischen Aufwand gegenüber dem Einsatz von z. B. pneumatisch betriebenen Membranpumpen als erste Fördereinrichtungen zu verringern.

Daneben kann es vorteilhaft möglich sein, auf einen leistungsfähigen Kompressor zum Antrieb von Pneumatik-Membranpumpen zur Förderung von Blut oder Dialysat zu verzichten. Dies kann vorteilhaft dazu beitragen eine - gegebenenfalls starke - Geräuschentwicklung zu minimieren.

Zudem kann es unter Verwenden von Dialysierflüssigkeit und/oder Substituatflüssigkeit als Arbeitsfluid vorteilhaft möglich sein, auf ein Aufbereiten eines weiteren Fluids zur Verwendung als steriles Arbeitsfluid, wie beispielsweise sterilisierte Luft, zu verzichten. Dialysierflüssigkeit und/oder Substituatflüssigkeit können entsprechend dem Stand der Technik on-line in der Behandlungsvorrichtung hergestellt und/oder aufbereitet werden.

Eine "offene" Schnittstelle zur Umgebung zum beispielsweise Ansaugen von Luft kann somit ferner vorteilhaft vermieden werden. Auf diese Weise kann es vorteilhaft möglich sein, den Aufwand beim Gewährleisten von Sicherheit und Hygiene zu reduzieren.

Da als strömende Medien vorzugsweise nicht komprimierbare Fluide eingesetzt werden können, kann es ferner vorteilhaft möglich sein, Strömungsgeräusche zu minimieren.

Im Folgenden wird die vorliegende Erfindung beispielhaft unter Bezugnahme auf die beigefügte Zeichnung beschrieben. Es gilt:
- Fig. 1: zeigt schematisch vereinfacht einen Aufbau eines hydraulischen Systems einer erfindungsgemäßen medizinischen Funktionseinrichtung;
- Fig. 2: zeigt einen Ausschnitt des Aufbaus der Fig. 1 mit Zu- bzw. Ableitung für ein Arbeitsfluid;
- Fig. 3: zeigt eine Doppelkolbenpumpe mit einem zweiten Arbeitsraum;
- Fig. 4: zeigt ein Fließbild mit modifizierter Ventilausstattung;
- Fig. 5: zeigt ein weiteres Fließbild mit modifizierter Ventilausstattung; und
- Fig. 6: zeigt einen Ausschnitt der Darstellung der Fig. 1 mit einer Einzelleitung.

Fig. 1 zeigt ein hydraulisches System 100 einer externen medizinischen Funktionseinrichtung als Beispiel einer medizinischen Funktionseinrichtung. Diese kann eine Blutkassette sein, wovon im Folgenden ausgegangen wird. Jedoch kann die externe medizinische Funktionseinrichtung auch etwas anderes als eine Blutkassette sein.

Das hydraulische System 100 weist ein erstes Fluidsystem 1 sowie ein zweites Fluidsystem 3 auf.

Das erste Fluidsystem 1 weist eine Vielzahl von ersten Fördereinrichtungen 51 (oder 51a), 53, 55 (oder 55a) und 57 auf. Diese sind rein beispielhaft als Membranpumpen 51, 53 und 55 und als Kolbenpumpe 57 ausgestaltet bzw. gezeigt.

Das zweite Fluidsystem 3 weist eine zweite Fördereinrichtung 7 auf. Die zweite Fördereinrichtung 7 kann als Zentrifugalpumpe ausgestaltet sein. In die externe medizinische Funktionseinrichtung integriert, d.h. kassettenintegriert, kann vorzugsweise ein innerhalb der geschlossenen Kassette magnetisch gelagerter und von außerhalb der Kassette magnetisch angetriebener Impeller sein. Dieser kann maschinenseitig von einer Ankoppelfläche oder -ebene einer Behandlungsvorrichtung angetrieben werden. Der Antrieb kann vorzugsweise kontaktlos erfolgen.

Die zweite Fördereinrichtung 7 fördert das Arbeitsfluid in einer ersten Förderrichtung. In Fig. 1 ist die erste Förderrichtung des Arbeitsfluids, welche im Uhrzeigersinn erfolgt, mit dem Dreiviertelkreis-förmigen Pfeil und den im zweiten Fluidsystem 3 dargestellten Pfeilspitzen angedeutet.

Wie in Fig. 1 gezeigt, ist ein Filter 9 oder eine Drossel im zweiten Fluidsystem 3 angeordnet.

Das zweite Fluidsystem 3 weist eine Druckleitung 11 auf, welche an einer Druckseite P+ der zweiten Fördereinrichtung 7 angeordnet ist.

Das zweite Fluidsystem 3 weist eine Saugleitung 13 auf, welche an einer Saugseite P- der zweiten Fördereinrichtung 7 angeordnet ist.

In Fig. 1 sind beispielhaft vier erste Fördereinrichtungen gezeigt. Die ersten Fördereinrichtungen können zwischen die Druckseite P+ und die Saugseite P- der zweiten Fördereinrichtung 7 geschaltet werden. Wie in Fig. 1 gezeigt, sind die ersten Fördereinrichtungen über abzweigende Leitungen 15 mit der Druckleitung 11 und der Saugleitung 13 verbunden. Die ersten Fördereinrichtungen 51 (oder 51a), 53, 55 (oder 55a) und 57 sind hier in Parallelschaltung mit der Druckseite P+ und der Saugseite P- der zweiten Fördereinrichtung 7 verbunden.

Die ersten Fördereinrichtungen 51 (oder 51a), 53, 55 (oder 55a) und 57 fördern das oder die medizinischen Fluide in einer der ersten Förderrichtung des Arbeitsfluids entgegengesetzten Richtung, also gegen den Uhrzeigersinn in Fig. 1, wie mit den in Fig. 1 gezeigten Pfeilspitzen angedeutet ist.

In dem hydraulischen System 100, wie es in Fig. 1 gezeigt ist, kann als erste Fördereinrichtung prinzipiell jede Pumpe Verwendung finden, die zum Betrieb eine Druckdifferenz ΔP benötigt. Werden z.B. Membranpumpen als erste Fördereinrichtungen 51, 51a, 53, 55 und 55a verwendet, so können im Betrieb über die Druckleitung 11 der zweiten Fördereinrichtung 7 jeweils die Arbeitsfluidkammern der Membranpumpen 51, 51a, 53, 55 und 55a gefüllt werden. Durch Umschalten entsprechender Ventile 17a auf der Eintritts-seite und 17b auf der Austrittsseite können die Arbeitsfluidkammern der Membranpumpen 51, 51a, 53, 55 und 55a wieder leer gesaugt werden.

Bei einer extrakorporalen Blutbehandlung mit Citrat-Antikoagulation (Ci-Ca-Antikoagulation) wird mindestens eine Membranpumpe 51 zur Förderung einer Citrat-haltigen Lösung und gleichzeitig mindestens eine weitere unabhängige Membranpumpe 51a zur Förderung einer Calcium-haltigen Lösung betrieben. Die Membranpumpe 53 kann beispielsweise dazu dienen, Heparin zu fördern. Die Membranpumpe 55 kann beispielsweise dazu dienen, Blut zu fördern. Die Membranpumpe 55a kann beispielsweise dazu dienen, Dialysat zu fördern. Alle der vorgenannten Pumpen 51, 51a, 53, 55 und 55a können gleichzeitig betrieben werden. Sie sind parallel zueinander geschaltet. Manche von ihnen könnten allerdings auch in Reihe zueinander geschaltet sein. Das hier Gesagte gilt jeweils auch für weitere, hier nicht dargestellte oder erwähnte Pumpen oder Membranpumpen.

Es kann auch eine Kolbenpumpe Verwendung finden, welche in Fig. 1 als Kolbenpumpe 57 dargestellt ist. In diesem Fall kann im Betrieb durch entsprechende Ventile ein Überdruck auf einen Antriebskolben 19 der Kolbenpumpe 57 gelenkt werden. Anders als beim Betrieb einer Membranpumpe können bei der Kolbenpumpe 57 die Pumpenhübe ausschließlich durch überdruck aus der Druckleitung 11 angetrieben werden.

Wie mit den Unterbrechungen der Leitungen 15 jeweils zwischen der Membranpumpe 53 und der Membranpumpe 55 angedeutet ist, kann eine beliebige weitere Anzahl an ersten Fördereinrichtungen im ersten Fluidsystem 1 angeordnet sein. Wenn die zweite Fördereinrichtung 7 im geschlossenen zweiten Fluidsystem 3 fördert, kann der Druck drehzahlabhängig ansteigen und das Fluid im Pumpengehäuse überströmen. Wenn die zweite Fördereinrichtung 7 in einem Kreislauf fördert, können sich auf der Druckseite P+ entsprechend hohe Drücke (bis ca. 2 bar) und auf der Saugseite P- Unterdrücke (bis ca. -800 mbar) ergeben. Daraus können Volumenströme mit bis zu 22 1/min resultieren.

Um Unterdruck zum Entleeren der ersten Fördereinrichtungen in ausreichender Menge vorhalten zu können, können auch mehrere Venturi-Düsen (in Fig. 1 nicht gezeigt) in das hydraulische System 100 eingeschaltet oder eingebaut werden.

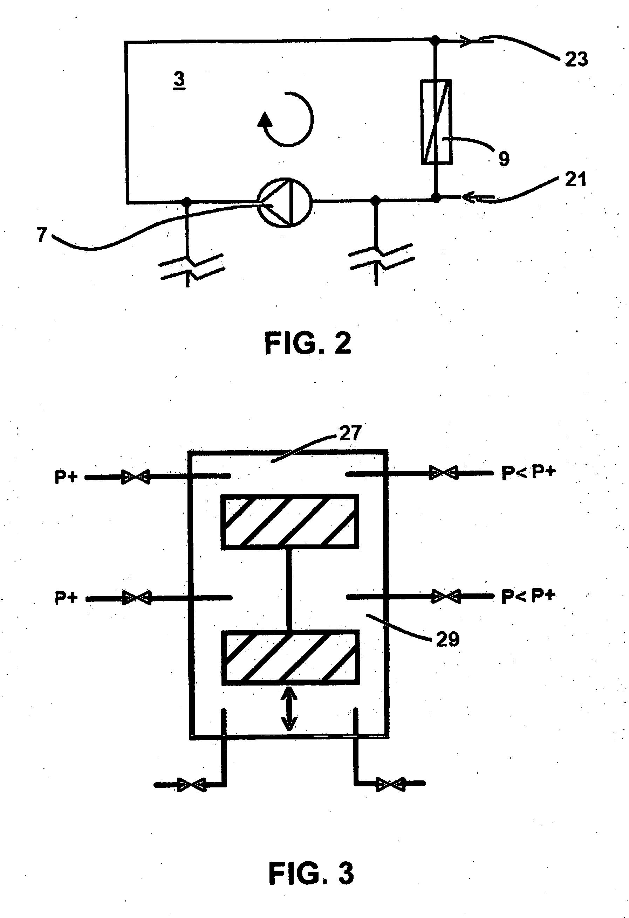

**Fig. 2** zeigt einen Ausschnitt des Aufbaus wie er in Fig. 1 dargestellt ist in einer erfindungsgemäßen Modifikation hiervon. Das in Fig. 2 dargestellte zweite Fluidsystem 3 weist vor bzw. nach dem Filter 9 (kann wie auch in Fig. 1 auch eine Drossel, ein Widerstand oder dergleichen sein) eine Zuleitung 21 und eine Ableitung 23 für ein Arbeitsfluid auf.

**Fig. 3** zeigt eine Doppelkolbenpumpe 25 mit einem ersten Arbeitsraum 27 und einem zweiten Arbeitsraum 29, insbesondere zur Verwendung als Pumpe 19 im ersten Fluidsystem 1.

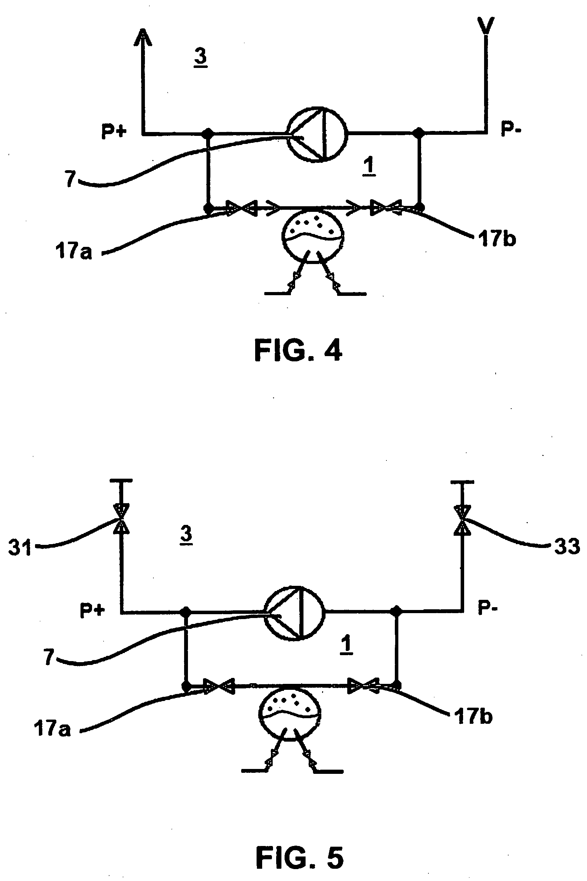

**Fig. 4** zeigt ein Fließbild mit modifizierter Ventilausstattung der erfindungsgemäßen medizinischen Funktionseinrichtung.

**Fig. 5** zeigt ein weiteres Fließbild mit modifizierter Ventilausstattung; gegenüber dem Aufbau der Fig. 4 sind in Fig. 5 weitere Ventile 31 und 33 dargestellt.

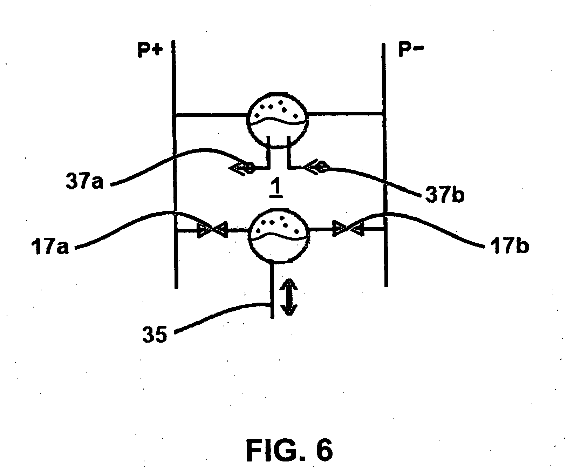

**Fig. 6** zeigt einen Ausschnitt der Darstellung der Fig. 1 mit einer Einzelleitung 35, mit welcher in einem von der vorliegenden Erfindung ebenfalls umfassten Sonderfall ein medizinisches Fluid mittels Stichleitung angesaugt und wieder zurückgepumpt werden kann. Diese Stichleitung bzw. Einzelleitung 35 kann ihren Einsatz finden bei der Probenentnahme, der batch-weisen Förderung, der Single-Needle-Hämodialyse, dem Peritonealdialyse-Cycler, usw.

In Fig. 6 sind ferner passive Rückschlageventile 37a und 37b gezeigt. Jedes der zuvor in beliebigem Zusammenhang erwähnten Ventile kann für den Fachmann erkennbar natürlich auch als ein solches Rückschlagventil 37a oder 37b ausgestaltet sein.

## Patentansprüche

1. Medizinische Funktionseinrichtung, mit wenigstens einem ersten Fluidsystem (1) zum Aufnehmen wenigstens eines medizinischen Fluids, wenigstens einer ersten Fördereinrichtung (51, 51a, 53, 55, 55a, 57) zum Fördern des medizinischen Fluids, wenigstens einem zweiten Fluidsystem (3) zum Aufnehmen wenigstens eines Arbeitsfluids, und wenigstens einer zweiten Fördereinrichtung (7) zum Antreiben der wenigstens ersten Fördereinrichtung, wobei die erste Fördereinrichtung (51, 51a, 53, 55, 55a, 57) angeordnet ist, um mittels des Arbeitsfluids betätigt zu werden, **dadurch gekennzeichnet, dass** die zweite Fördereinrichtung (7) als Zentrifugalpumpe oder als Kreiselpumpe ausgestaltet ist.

2. Medizinische Funktionseinrichtung nach Anspruch 1, wobei wenigstens eine erste Fördereinrichtung (51, 51a, 53, 55, 55a, 57) mit einer Druckseite (P+) und/oder einer Saugseite (P-) der zweiten Fördereinrichtung (7) verbunden oder verbindbar angeordnet ist.

3. Medizinische Funktionseinrichtung nach Anspruch 2, wobei erste Fördereinrichtungen (51, 51a, 53, 55, 55a, 57) in Parallelschaltung mit der Druckseite (P+) und/oder der Saugseite (P-) der zweiten Fördereinrichtung (7) verbunden oder verbindbar angeordnet sind.

4. Medizinische Funktionseinrichtung nach Anspruch 2, wobei erste Fördereinrichtungen (51, 51a, 53, 55, 55a, 57) in Reihenschaltung mit der Druckseite (P+) und/oder der Saugseite (P-) der zweiten Fördereinrichtung (7) verbunden oder verbindbar angeordnet sind.

5. Medizinische Funktionseinrichtung nach einem der vorangegangenen Ansprüche, wobei die erste Fördereinrichtung (51, 51a, 53, 55, 55a, 57) als Verdrängerpumpe ausgestaltet ist, die ausgewählt ist aus Membranpumpen, Kolbenpumpen, Schlauchpumpen und peristaltische Pumpen.

6. Medizinische Furiktionseinrichtung nach einem der vorangegangenen Ansprüche, wobei die zweite Fördereinrichtung (7) wenigstens einen Rotationsabschnitt aufweist, welcher magnetisch gelagert ist.

7. Medizinische Funktionseinrichtung nach Anspruch 6, wobei der Rotationsabschnitt konfiguriert ist, um mittels eines externen Antriebs magnetisch antreibbar zu sein.

8. Medizinische Funktionseinrichtung nach einem der vorangegangenen Ansprüche, wobei das erste Fluidsystem (1) und das zweite Fluidsystem (3) fluiddicht voneinander getrennt ausgestaltet sind.

9. Medizinische Funktionseinrichtung nach einem der vorangegangenen Ansprüche, wobei das medizinische Fluid ausgewählt ist aus Blut, Dialysierflüssigkeit, Substituatflüssigkeit, Medikamenten, Medikamentenzubereitungen sowie Mischungen oder Kombinationen derselben.

10. Medizinische Funktionseinrichtung nach einem der vorangegangenen Ansprüche, wobei das Arbeitsfluid ausgewählt ist aus Dialysierflüssigkeit, Substituatflüssigkeit, steriler Druckluft, Hydrauliköl, Wasser, sowie Mischungen oder Kombinationen derselben.

11. Medizinische Funktionseinrichtung nach einem der vorangegangenen Ansprüche, welche als Disposable-Kassette, insbesondere als Blutkassette und/oder als Dialysatkassette für die extrakorporale Blutbehandlung oder als Kassette für die Peritonealdialyse ausgestaltet ist.

12. Behandlungsvorrichtung zum Behandeln medizinischer Fluide, welche wenigstens eine Steuer- oder Regeleinrichtung aufweist, die dazu vorgesehen und konfiguriert ist, um wenigstens die zweite Fördereinrichtung (7) einer medizinischen Funktionseinrichtung gemäß einem der Ansprüche 1 bis 11 anzutreiben, wobei
- die Behandlungsvorrichtung zur Blutbehandlung mittels Hämodialyse, Hämofiltration, Hämodiafiltration oder zur Peritonealdialyse oder zur Mehrfachinfusionstechnik geeignet und vorgesehen ist, oder wobei
- die Steuer- oder Regeleinrichtung konfiguriert ist, Ventile im ersten und/oder im zweiten Fluidsystem mittels geeigneter Aktoren zu steuern und/oder zu regeln.

13. Behandlungsvorrichtung nach Anspruch 12, mit einer Einrichtung, die dazu vorgesehen und konfiguriert ist, die zweite Fördereinrichtung (7) über eine magnetische Antriebsschnittstelle anzutreiben.

14. Behandlungsvorrichtung nach Anspruch 12 oder 13, welche funktionell mit einer medizinischen Funktionseinrichtung gemäß einem der Ansprüche 1 bis 11 koppelbar ist oder eine solche aufweist.

15. Behandlungsvorrichtung nach einem der Ansprüche 12 bis 14, welche als Blutbehandlungs- oder Infusionsvorrichtung ausgestaltet ist.

## Claims

1. A medical functional device comprising at least one first fluid system (1) for receiving at least one medical fluid, at least one first conveying means (51, 51a, 53, 55, 55a, 57) for conveying the medical fluid, at least a second fluid system (3) for receiving at least a working fluid, and at least a second conveying means (7) for driving the at least one first conveying means, wherein the first conveying means (51, 51a, 53, 55, 55a, 57) is arranged to be actuated by means of the working fluid,
**characterized in that**:
the second conveying means (7) is embodied as a centrifugal pump or as a rotary pump.

2. The medical functional device according to claim 1, wherein at least one first conveying means (51, 51a, 53, 55, 55a, 57) is arranged to be connected or connectable with a pressure side (P+) and/or with a suction side (P-) of the second conveying means (7).

3. The medical functional device according to claim 2, wherein first conveying means (51, 51a, 53, 55, 55a, 57) are arranged to be connected or connectable in parallel with the pressure side (P+) and/or with the suction side (P-) of the second conveying means (7).

4. The medical functional device according to claim 2, wherein first conveying means (51, 51a, 53, 55, 55a, 57) are arranged to be connected or connectable in series with the pressure side (P+) and/or with the suction side (P-) of the second conveying means (7).

5. The medical functional device according to anyone of the preceding claims, wherein the first conveying means (51, 51a, 53, 55, 55a, 57) is embodied as a positive displacement pump, which is selected from diaphragm pumps, piston pumps, hose pumps and peristaltic pumps.

6. The medical functional device according to anyone of the preceding claims, wherein the second conveying means (7) comprises at least one rotating section, which is magnetically born.

7. The medical functional device according to claim 6, wherein the rotating section is configured to be magnetically drivable by means of an external driver.

8. The medical functional device according to anyone of the preceding claims, wherein the first fluid system (1) and the second fluid system (3) are configured to be fluid-tightly separated from each other.

9. The medical functional device according to anyone of the preceding claims, wherein the medical fluid is selected from blood, dialysis fluid, substitute fluid, drugs, drug preparations, as well as mixtures or combinations thereof.

10. The medical functional device according to anyone of the preceding claims, wherein the working fluid is selected from dialysis fluid, substitute fluid, sterile compressed air, hydraulic oil, water, as well as mixtures or combinations thereof.

11. The medical functional device according to anyone of the preceding claims, embodied as a disposable cassette, in particular as a blood cassette and/or as a dialysate cassette for the extracorporeal blood treatment or as a cassette for peritoneal dialysis.

12. A treatment apparatus for treating medical fluids, comprising at least one control or regulation device provided or configured for driving at least the second conveying means (7) of a medical functional device according to anyone of claims 1 to 11, wherein
- the treatment apparatus is adapted and provided for blood treatment by means of hemodialysis, hemofiltration, hemodiafiltration or for peritoneal dialysis or multiple infusions technique, or wherein
- the control or regulation device is configured for controlling and/or regulating valves in the first and/or the second fluid system by means of suitable actuators.

13. The treatment apparatus according claim 12 comprising a device provided and configured for driving the second conveying means (7) through a magnetic drive interface.

14. The treatment apparatus according claim 12 or 13 functionally coupleable with a medical functional device according to anyone of claims 1 to 11 or comprising such a device.

15. The treatment apparatus according claim 12 to 14 embodied as a blood treatment apparatus or as an infusion apparatus.

## Revendications

1. Un dispositif fonctionnel médical comprenant au moins un premier système fluidique (1) destiné à recevoir au moins un fluide médical, au moins un premier moyen de transport (51, 51a, 53, 55, 55a, 57) pour acheminer le fluide médical, au moins un second système fluidique (3) destiné à recevoir au moins un fluide de travail, et au moins un second moyen de transport (7) destiné à actionner au moins le premier moyen de transport, où le premier moyen de transport (51, 51a, 53, 55, 55a, 57) est agencé de façon à être actionné via le fluide de travail,
**caractérisé en ce que**:
le second moyen de transport (7) est configuré comme une pompe centrifuge ou une pompe rotative.

2. Le dispositif fonctionnel médical selon la première revendication, où au moins un premier moyen de transport (51, 51a, 53, 55, 55a, 57) est agencé de façon à être connecté ou connectable à un côté pression (P+) et/ou à un côté aspiration (P-) du second moyen de transport (7).

3. Le dispositif fonctionnel médical selon la deuxième revendication, où des premiers moyens de transport (51, 51a, 53, 55, 55a, 57) sont agencés en parallèle de façon à être connectés ou connectables avec un côté pression (P+) et/ou avec un côté aspiration (P-) du second moyen de transport (7).

4. Le dispositif fonctionnel médical selon la deuxième revendication, où des premiers moyens de transport (51, 51a, 53, 55, 55a, 57) sont agencés en série de façon à être connectés ou connectables avec un côté pression (P+) et/ou avec un côté aspiration (P-) du second moyen de transport (7).

5. Le dispositif fonctionnel médical selon l'une quelconque des revendications précédentes, où le premier moyen de transport (51, 51a, 53, 55, 55a, 57) est formé comme pompe volumétrique choisie parmi les pompes à membrane, les pompes à piston, les pompes tubulaires et les pompes péristaltiques.

6. Le dispositif fonctionnel médical selon l'une quelconque des revendications précédentes, où le second moyen de transport (7) comprend au moins une section rotative supportée magnétiquement.

7. Le dispositif fonctionnel médical selon la revendication 6, où la section rotative est configurée de façon à pouvoir être entraînée magnétiquement au moyen d'un entraînement externe.

8. Le dispositif fonctionnel médical selon l'une quelconque des revendications précédentes, où le premier système fluidique (1) et le second système fluidique (3) sont formés de façon à être séparés l'un de l'autre de manière étanche.

9. Le dispositif fonctionnel médical selon l'une quelconque des revendications précédentes, où le fluide médical est choisi parmi le sang, le liquide de dialyse, le liquide de substitution, les médicaments, les préparations de médicaments, ainsi que les mélanges ou les combinaisons de ceux-ci.

10. Le dispositif fonctionnel médical selon l'une quelconque des revendications précédentes, où le fluide de travail est choisi parmi le liquide de dialyse, le liquide de substitution, l'air comprimé stérile, l'huile hydraulique, l'eau, ainsi que les mélanges ou les combinaisons de ceux-ci.

11. Le dispositif fonctionnel médical selon l'une quelconque des revendications précédentes, formé comme cassette jetable, notamment comme cassette à sang et/ou comme cassette à dialysat pour le traitement extracorporel du sang ou comme cassette pour la dialyse péritonéale.

12. Un appareil de traitement destiné à traiter des fluides médicaux, comprenant au moins un système de commande ou de régulation prévu et configuré pour au moins actionner le second dispositif de transport (7) d'un dispositif fonctionnel médical selon l'une des revendications 1 à 11, où
- l'appareil de traitement est adapté et prévu pour le traitement du sang au moyen d'une hémodialyse, d'une hémofiltration, d'une hémodiafiltration ou pour la dialyse péritonéale ou pour la technique de perfusion multiple, ou bien où
- le système de commande ou de régulation est configuré de façon à commander et/ou à réguler des vannes dans le premier et/ou dans le second système fluidique au moyen d'actionneurs adaptés.

13. L'appareil de traitement selon la revendication 12 comprenant un dispositif prévu et configuré pour actionner le second dispositif de transport (7) via une interface d'actionnement magnétique.

14. L' appareil de traitement selon la revendication 12 ou 13, pouvant être couplé de manière fonctionnelle avec un dispositif fonctionnel médical selon l'une des revendications 1 à 11 ou comprenant un tel dispositif.

15. L' appareil de traitement selon l'une des revendications 12 à 14 formé comme un appareil de traitement du sang ou comme un appareil de perfusion.
